# EUROPEAN PATENT SPECIFICATION

(11) **EP 1 422 278 B1**
(45) Date of publication and mention of the grant of the patent: **07.06.2006**
(21) Application number: 03026061.6
(22) Date of filing: 12.11.2003
(51) Int. Cl.: C09J 7/02, A61L 15/26

(54) **Pressure-sensitive adhesive sheet for medical use and process for producing the same**
Druckempfindliche Klebefolie für medizinische Zwecke und Verfahren zu deren Herstellung
Feuille adhésive sensible à la pression à usage médical et son procédé de préparation

(30) Priority: 20.11.2002 JP 2002336645
(43) Date of publication of application: 26.05.2004
(73) Proprietor: NITTO DENKO CORPORATION, Osaka (JP)
(72) Inventor: Yamamoto, Yuko, Nitto Denko Corpor., Osaka (JP); Furumori, Kenji, Nitto Denko Corpor., Osaka (JP); Yoshikawa, Toshiyuki, Nitto Denko Corpor., Osaka (JP)
(74) Representative: Grünecker, Kinkeldey, Stockmair & Schwanhäusser Anwaltssozietät

(56) References cited:
- DE-A- 19 913 719
- PATENT ABSTRACTS OF JAPAN vol. 2000, no. 12, 3 January 2001 (2001-01-03) & JP 2000 248236 A (NITTO DENKO CORP), 12 September 2000 (2000-09-12)
- DATABASE WPI Section Ch, Week 200326 Derwent Publications Ltd., London, GB; Class A14, AN 2003-260467 XP002271621 & JP 2002 322359 A (NITTO DENKO CORP), 8 November 2002 (2002-11-08)

## Description

The present invention relates to a pressure-sensitive adhesive sheet for use in the field of medical and sanitary materials. More particularly, the invention relates to a medical pressure-sensitive adhesive sheet to be used in applications such as first-aid plasters, large plasters, pressure-sensitive adhesive bandages, and dressing materials.

Pressure-sensitive adhesive sheets intended to be applied to the skin or the like have been produced by applying an organic-solvent-based pressure-sensitive adhesive to a substrate, e.g., a plastic film, and drying the adhesive. However, with the recent growing concern about environmental problems, the conventional organic-solvent-based pressure-sensitive adhesives are being shifted to solvent-free pressure-sensitive adhesives such as the emulsion type, hot-melt type, and radiation-curing type. Although attempts are being made to put these solventless pressure-sensitive adhesives, which have their own features, to practical use, it is never easy to realize, by using any of these solvent-free pressure-sensitive adhesives, a pressure-sensitive adhesive sheet which is equal in performance to those obtained with organic-solvent-based pressure-sensitive adhesives. In particular, with respect to emulsion type pressure-sensitive adhesives, there are cases where the surfactants contained therein bleed out after application and drying, causing various adverse influences.

When satisfactory anchoring is not obtained at the boundary between the substrate and the adhesive layer, application of this pressure-sensitive adhesive sheet to an adherend and later stripping thereof may result in a trouble in which the pressure-sensitive adhesive partly peels off the substrate and remains on the adherend. Namely, the so-called adhesive remaining occurs. For example, when application of a sticking plaster to the skin and later stripping thereof cause the adhesive remaining, dust particles and the like may adhere to the pressure-sensitive adhesive remaining on the skin and act as an infection source on the wound. For securing anchoring to a substrate, the substrate may be coated beforehand to form a primer layer.

In the case where a primer is used for eliminating the problem of adhesive remaining, satisfactory anchoring is realized only when a proper primer is selected. It is, however, difficult to select a proper primer. Furthermore, additionally conducting a priming step leads to a cost increase. There has hence been a desire for a production process which does not necessitate a priming step.

In medical pressure-sensitive adhesive sheets such as first-aid plasters and rolled plasters, films comprising a plasticized poly(vinyl chloride) as the main component and formed by calendering, casting, or another technique have hitherto been used as substrates. Films comprising a plasticized poly(vinyl chloride) have such satisfactory stress relaxation characteristics that they show a high stress in the beginning of stretching but, when kept in the stretched state, abruptly come to have a relaxed tensile stress with the lapse of time. Use of a pressure-sensitive adhesive sheet employing a film having such satisfactory stress relaxation characteristics as the substrate produces an excellent effect that after application to the skin, the tensile stress is gradually relaxed to reduce the burden on the skin. However, use of the plasticized poly(vinyl chloride) requires measures against adverse influences due to liquid phthalate plasticizers and the chlorine contained in poly(vinyl chloride). In addition, flexible poly(vinyl chloride) generally contain a large amount of a liquid plasticizer in order to have flexibility, and this plasticizer migrates into the pressure-sensitive adhesive to reduce the cohesive force of the pressure-sensitive adhesive. It has been pointed out that this leads to adhesive remaining and a decrease in adhesive force. Thus, there has been a desire for a material which can replace poly(vinyl chloride).

For example, JP-A-2000-230115 discloses a process for forming a film from an aqueous urethane-acrylic composite dispersion. This dispersion is a solvent-free emulsion of a non-vinyl chloride-type self-emulsified polymer and the film can be regulated so as to have any desired properties by regulating the urethane/acrylic proportion and composition. This film is hence suitable for use as the substrate of a pressure-sensitive adhesive sheet. Furthermore, since this substrate has satisfactory conformability to the skin, the pressure-sensitive adhesive sheet, after application, for example, as a plaster to a finger or the like, does not suffer peeling or the like upon bending. JP-A-2000-248236 discloses a pressure-sensitive adhesive sheet comprising a substrate and a pressure-sensitive adhesive layer which are respectively a coating film formed from an aqueous polymer dispersion. The performances (properties) of this pressure-sensitive adhesive sheet as a whole can be easily regulated.

DE-A-19913719 discloses an adhesive sheet comprising a supporting layer (A) on the basis of polyacrylate, and one or both sides thereof an adhesive layer (B). The supporting layer (A) and the adhesive layer (B) are formed separately and then laminated to each other.

However, those pressure-sensitive adhesive sheets according to related-art techniques have had the following problems. Immediately after laminating of the pressure-sensitive adhesive layer to the substrate, this pressure-sensitive adhesive layer is in an insufficiently anchored state and is apt to peel off the substrate. Accordingly, this pressure-sensitive adhesive sheet cannot be processed immediately after the laminating. Furthermore, when a pressure-sensitive adhesive containing a large amount of a plasticizer or a natural rubber latex is used, a reduced anchoring effect is apt to result.
Document 1:
   JP-A-2000-230115
Document 2:
   JP-A-2000-248236

It was the object of the invention to provide a pressure-sensitive adhesive sheet for medical use which can realize satisfactory anchoring and can be produced without using a primer, which is required to be properly selected, or without requiring a priming step, which leads to cost increase, and further to provide processes for producing this pressure-sensitive adhesive sheet for medical use. This object is achieved by the processes of claims 1 and 2 and further by the pressure-sensitive addesive sheet of claim 3. Preferred embodiments are set forth in the subclaims.

The present invention provides a pressure-sensitive adhesive sheet for medical use, which comprises a supporting layer and a pressure-sensitive adhesive layer provided on at least one side of the supporting layer, wherein each of the supporting layer and the pressure-sensitive adhesive layer is prepared from an aqueous polymer dispersion and the supporting layer and the pressure-sensitive adhesive layer are in the state of being anchored each other at the boundary thereof, obtainable by the processes of the present invention.

In this pressure-sensitive adhesive sheet for medical use, the aqueous polymer dispersion used for forming the supporting layer preferably is an aqueous urethane-acrylic composite dispersion.

The aqueous polymer dispersion used for forming the supporting layer is preferably that can give, after application and drying, gives a coating film having a 100% modulus of from 5 N/mm² to 15 N/mm².

Furthermore, the aqueous polymer dispersion used for forming the supporting layer is preferably that can give, after application and drying, a coating film having a stress relaxation time of 50 seconds or shorter.

The invention further provides a process for producing a pressure-sensitive adhesive sheet for medical use, which comprises simultaneously applying at least a coating fluid for forming a pressure-sensitive adhesive layer and a coating fluid for forming a supporting layer in this order to a releasability-imparted side of a release member and then drying the coating fluids to form a multilayer structure comprising a pressure-sensitive adhesive layer and a supporting layer which are in the state of being anchored each other at the boundary thereof.

The invention furthermore provides a process for producing a pressure-sensitive adhesive sheet for medical use, which comprises applying at least a coating fluid for forming a pressure-sensitive adhesive layer to a releasability-imparted side of a release member, subsequently applying a coating fluid for forming a supporting layer on the pressure-sensitive adhesive layer before the pressure-sensitive adhesive layer dries, and then drying the whole to thereby form a multilayer structure comprising a supporting layer and a pressure-sensitive adhesive layer which are in the state of being anchored each other at the boundary thereof.

By way of example and to make the description more clear, reference is made to the accompanying drawing in which:
Fig. 1 is an electron photomicrograph showing a section of a pressure-sensitive adhesive sheet for medical use according to the present invention.
Fig. 2 is an electron photomicrograph showing a section of a comparative pressure-sensitive adhesive sheet for medical use.

According to the invention, the pressure-sensitive adhesive sheet has, at the boundary between the supporting layer and the pressure-sensitive adhesive layer, an area in which the aqueous polymer dispersions respectively used for forming the two layers were mixed with each other. Therefore, satisfactory anchoring can be realized without requiring forming of a primer layer. Furthermore, since an priming step is not required, a cost reduction can be achieved.

The pressure-sensitive adhesive sheet for medical use according to the present invention is a sheet which comprises a supporting layer and a pressure-sensitive adhesive layer provided on at least one side of the supporting layer, and wherein each of the supporting layer and the pressure-sensitive adhesive layer is a layer formed from an aqueous polymer dispersion. The supporting layer and the pressure-sensitive adhesive layer are in the state of being anchored each other at the boundary between them. The term "state of being anchored each other" as used herein means that after the aqueous polymer dispersion for forming a pressure-sensitive adhesive layer and the aqueous polymer dispersion for forming a supporting layer are applied and dried, the two polymers at the boundary are in the state of being mingled with each other, in the state of being slightly included in each other, in the state of being partly interpenetrated, or in a similar state. Since the pressure-sensitive adhesive layer and supporting layer in the pressure-sensitive adhesive sheet for medical use of the present invention are in the state of being thus anchored each other at the boundary between them, satisfactory anchoring is exhibited even immediately after application and drying. Accordingly, the problem of adhesive remaining does not arise.

The thickness of the layer at which the supporting layer and the pressure-sensitive adhesive layer are mingled with each other may be 5 µm or less, preferably 3 µm or less, more preferably 1 µm or less. The thicknesss exceeding 5 µm may cause adverse effects against the film properties of the supporting layer and the adhesive properties of the pressure-sensitive adhesive layer. The thickness is preferably 0.001 µm or more.

The term "film" as used in the present invention means a conception including a sheet, while the term "sheet" means a conception including a film.

The aqueous polymer dispersion to be used for forming a supporting layer (hereinafter often referred to as "aqueous polymer dispersion for forming a supporting layer) is one which, when applied and dried, gives a coating film having no tackiness and having a certain degree of 100% modulus and stress relaxation characteristics.

According to the present invention, the 100% modulus of the coating film is preferably from 5 N/mm² to 15 N/mm² , more preferably from 5 N/mm² to 10 N/mm². The term "100% modulus" herein means a force required for the 100% elongation of the film. In case where the 100% modulus thereof is lower than 5 N/mm ², the film has tackiness and poor stiffness and is difficult to handle. In case where the 100% modulus thereof is higher than 15 N/mm², the film shows reduced conformability to the skin and gives a poor stiff feeling in wear.

With respect to stress relaxation characteristics, the shorter the stress relaxation time, the better the stress relaxation characteristics. According to the present invention, the stress relaxation time is preferably 50 seconds or shorter. The term "stress relaxation time" as used herein means the time period required for the stress of the film kept in a 50%-elongated state to decrease to 1/e (e=2.7183) the initial value (stress at 50% elongation).

The aqueous polymer dispersion to be used for forming a supporting layer is an aqueous polymer dispersion having the properties described above. The dispersion may further contain, when needed, a crosslinking agent, filler, pigment, antioxidant, ultraviolet absorber, and other ingredients in order to regulate 100% modulus or strength.

Examples of the polymer contained in the aqueous polymer dispersion include synthetic rubber polymers such as poly(styrene-butadiene) polymers and poly(acrylonitrile-butadiene) polymers, condensation polymers such as polyurethane polymers, polyepoxy polymers, and polyester polymers, acrylic polymers, and polystyrene polymers. When any of these polymers is used to produce an aqueous polymer dispersion, it is necessary to use an emulsifying agent during the synthesis thereof or emulsification thereof in water.

According to the present invention, an aqueous urethane-acrylic composite dispersion known as an aqueous dispersion of a composite of a urethane prepolymer with an acrylic polymer may be used as the polymer for constituting the aqueous polymer dispersion. The aqueous urethane-acrylic composite dispersion can be synthesized even without an emulsifying agent. This dispersion gives a supporting layer having satisfactory water resistance and excellent anchoring to the pressure-sensitive adhesive layer. Furthermore, property design of this dispersion can be changed easily. Accordingly, the composite dispersion is especially preferred.

The term "composite" as used herein does not mean a mere mixture of heterogeneous polymers which causes a phase separation but means a mixture wherein the polymers are mixed in micrometer orders and shows a macroscopically homogeneous phase due the the interactions between the polymers (i.e., a so-called "polymer alloy" condition). The composite shows average physical properties between the polymers and also has new physical properties.

A method for obtaining an aqueous urethane-acrylic composite dispersion will be explained below. Namely, a) an aqueous urethane-acrylic composite dispersion is prepared, and b) one or more nontackiness-imparting monomers comprising an alkyl (meth)acrylate as a major component and giving a polymer having a glass transition temperature of 273 K or higher are added to the dispersion and polymerization is carried out, whereby an aqueous dispersion of a urethane-acrylic polymer which is not tacky at room temperature can be produced.

In step a, an aqueous urethane-acrylic composite dispersion can be prepared by a method (1) which comprises: mixing a carboxyl-containing urethane prepolymer synthesized using a polyol and a polyisocyanate with one or more monomers comprising an alkyl (meth)acrylate as a major component; neutralizing the carboxyl groups of the carboxyl-containing urethane prepolymer and dispersing this prepolymer in water; extending the main chain of the carboxyl-containing urethane prepolymer by reacting the isocyanate groups; and polymerizing the monomers. Alternatively, the composite dispersion may be prepared by the following method (2). A polymer having carboxyl groups and hydroxyl groups in the molecule obtained by copolymerizing a monomer mixture comprising an alkyl (meth)acrylate as a major component and a carboxyl-containing monomer, which is an acrylic ingredient, is mixed with a polyol. This mixture is reacted with a polyisocyanate to synthesize an isocyanate prepolymer. The carboxyl groups of the isocyanate prepolymer are neutralized and this prepolymer is dispersed in water. The main chain of this prepolymer is extended by reacting the isocyanate groups to thereby produce an aqueous urethane-acrylic composite dispersion.

The polyol to be used here for constituting a urethane desirably is one having two or more hydroxyl groups per molecule. Examples of low-molecular weight polyols include dihydric alcohols such as ethylene glycol, diethylene glycol, propylene glycol, butylene glycol, and hexamethylene glycol and trihydric or tetrahydric alcohols such as trimethylolpropane, glycerol, and pentaerythritol.

Examples of high-molecular weight polyols include polyether polyols, polyester polyols, acrylic polyols, and epoxy polyols. Examples of the polyether polyols include polyethylene glycol, polypropylene glycol, and polytetramethylene glycol. Examples of the polyester polyols include products of the polycondensation of an alcohol such as, e.g., any of the dihydric alcohols mentioned above, dipropylene glycol, 1,4-butanediol, 1,6-hexanediol, or neopentyl glycol with a dibasic acid such as, e.g., adipic acid, azelaic acid, or sebacic acid. Other examples thereof include polyol polycarbonate diols formed by the ring-opening polymerization of a lactone compound, such as polycaprolactone. Examples of the acrylic polyols include copolymers of a monomer having a hydroxyl group, such as hydroxyethyl (meth)acrylate or hydroxypropyl (meth)acrylate, and copolymers of a hydroxyl-containing compound and an acrylic monomer. Examples of the epoxy polyols include amine-modified epoxy resins.

Those polyols may be used alone or in combination of two or more thereof.

Examples of the polyisocyanate to be used for constituting a urethane include aromatic, aliphatic, and alicyclic diisocyanates and the dimers, trimers, and the like of these diisocyanates. Examples of the aromatic, aliphatic, and alicyclic diisocyanates include tolylene diisocyanate, diphenylmethane diisocyanate, hexamethylene diisocyanate, xylylene diisocyanate, hydrogenated xylylene diisocyanate, isophorone diisocyanate, hydrogenated diphenylmethane diisocyanate, 1,5-naphthylene diisocyanate, 1,3-phenylene diisocyanate, 1,4-phenylene diisocyanate, butane 1,4-diisocyanate, 2,2,4-trimethylhexamethylene diisocyanate, 2,4,4-trimethylhexamethylene diisocyanate, cyclohexane 1,4-diisocyanate, dicyclohexylmethane 4,4-diisocyanate, 1,3-bis(isocyanatomethyl)cyclohexane, methylcyclohexane diisocyanate, and m-tetramethylxylylene diisocyanate. Also usable are the dimers and trimers of these isocyanates and polyphenylmethane polyisocyanates. Examples of the trimers include the isocyanurate type, biuret type, and allophanate type; and such trimers may be used according to need.

Those polyisocyanates can be used alone or in combination of two or more thereof. From the standpoints of rapid reaction with the polyol and of inhibiting reaction with water, it is preferred to use an alicyclic diisocyanate.

The amounts of the polyol and polyisocyanate to be used for forming a urethane polymer are not particularly limited. However, the amount of the polyol to be used relative to the polyisocyanate amount is, for example, preferably 0.8 or larger, more preferably from 0.8 to 4.0, and most preferably from 0.8 to 3.0, in terms of NCO/OH (equivalent ratio). In case where NCO/OH is smaller than 0.8, a urethane polymer having a sufficiently extended molecular chain cannot be obtained and film strength and elongation are apt to decrease. As long as NCO/OH is 3.0 or smaller, flexibility can be sufficiently secured.

A catalyst may be used in the reaction of the isocyanate with the hydroxyl groups of the polyol. For example, a catalyst in general use for urethane-forming reactions may be used, such as dibutyltin dilaurate, tin octoate, or 1,4-diazabicyclo[2.2.2]octane.

The alkyl (meth)acrylate is preferably one in which the alkyl group has 1 to 14 carbon atoms. Examples of the carboxyl-containing monomer include (meth)acrylic acid, maleic acid, and itaconic acid.

The nontackiness -imparting monomers to be used in step b preferably are ones which comprise an alkyl (meth)acrylate as a major component and give a polymer having a glass transition temperature of 273 K or higher, preferably 300 K or higher. Other copolymerizable acrylic monomer ingredients may also be used.

The proportion of the aqueous urethane-acrylic composite dispersion to be mixed is preferably such that the amount of the aqueous urethane-acrylic composite dispersion is from 20 to 90% by weight on a solid basis and that of the nontackiness-imparting monomers is from 80 to 10% by weight. The proportion thereof is preferably regulated so as to finally result in a polyol unit content of from 10 to 50% by weight, polyisocyanate unit content of from 2 to 20% by weight, and acrylic unit content of from 40 to 90% by weight. By suitably selecting the kinds of the ingredients, combination thereof, etc. and regulating the proportions thereof in such a manner, a coating film (supporting layer) having a 100% modulus of from 5 N/mm² to 15 N/mm² and a stress relaxation time of 50 seconds or shorter can be realized.

The aqueous polymer dispersion to be used for forming a pressure-sensitive adhesive layer (hereinafter often referred to as "aqueous polymer dispersion for forming a pressure-sensitive adhesive layer) will be explained next.

The aqueous polymer dispersion to be used for forming a pressure-sensitive adhesive layer is an aqueous polymer dispersion which, when applied and dried, gives a film which shows pressure-sensitive adhesive properties and is satisfactory in adhesive force, holding power, etc. According to the present invention, additives such as a crosslinking agent, tackifier resin, filler, and pigment may be incorporated when necessary. Examples of the aqueous polymer dispersion for forming a pressure-sensitive adhesive layer include aqueous dispersions of an acrylic polymer or urethane-acrylic polymer and aqueous dispersions of a rubber polymer such as a synthetic rubber, e.g., a styrene/butadiene copolymer, or natural rubber.

The aqueous dispersions of an acrylic polymer can be produced by mixing an alkyl (meth)acrylate as a major ingredient with, e.g., a carboxyl-containing monomer and emulsion-polymerizing the monomers in an ordinary way.

The aqueous dispersions of a urethane-acrylic polymer may be prepared in the same manner as the method for preparing the aqueous urethane-acrylic composite dispersion to be used for forming a supporting layer. Namely, an aqueous dispersion containing a urethane-acrylic polymer evenly dispersed in the water can be obtained by the method (1) or (2) in the step a described above.

The pressure-sensitive adhesive sheet of the present invention is produced using an aqueous polymer dispersion for forming a supporting layer and an aqueous polymer dispersion for forming a pressure-sensitive adhesive layer.

Specifically, the adhesive sheet can be produced by the following process (A). A release film which has undergone a releasability-imparting treatment (release member) is coated, on the releasability-imparted side, with an aqueous polymer dispersion for forming a pressure-sensitive adhesive layer. Subsequently, an aqueous polymer dispersion for forming a supporting layer is applied thereto before the resulting pressure-sensitive adhesive layer dries. When needed, an aqueous polymer dispersion for forming a pressure-sensitive adhesive layer is further applied to this layer. Thereafter, the whole is dried. Thus, a pressure-sensitive adhesive sheet comprising a supporting layer and a pressure-sensitive adhesive layer provided on one side or both sides thereof can be produced. The surface of the pressure-sensitive adhesive layer can be protected by keeping the release film adherent thereto until use.

Alternatively, the following process (B) can be used. An aqueous polymer dispersion for forming a pressure-sensitive adhesive layer and an aqueous polymer dispersion for forming a supporting layer are simultaneously applied to the releasability-imparted side of a release film which has undergone a releasability-imparting treatment (release member). When needed, an aqueous polymer dispersion for forming a pressure-sensitive adhesive layer is further applied simultaneously on the aqueous polymer dispersion for forming a supporting layer. Thereafter, the whole is dried. Thus, a pressure-sensitive adhesive sheet comprising a supporting layer and a pressure-sensitive adhesive layer disposed on one or each side thereof can be produced. The surface of the pressure-sensitive adhesive layer can be protected by keeping the release film adherent thereto until use.

According to the present invention, processes (A) and (B) may be used in combination. For example, it is possible to use a method which comprises simultaneously applying an aqueous polymer dispersion for forming a pressure-sensitive adhesive layer and an aqueous polymer dispersion for forming a supporting layer to a release film, subsequently applying an aqueous polymer dispersion for forming a pressure-sensitive adhesive layer on the supporting layer before these layers dry, and then drying the whole layers to thereby produce a pressure-sensitive adhesive sheet for medical use.

A coating process suitable for process (A) is to use, e.g., a die coater so that a given amount of a coating fluid can be applied on a lower coating layer before this layer dries. A coating technique suitable for process (B) is to use, e.g., a die coater having two or more manifolds so as to apply two or more layers each in a laminar flow state.

With respect to drying conditions, it is preferred to suitably select conditions which do not cause foaming, cracking, or the like to the coating film and to dry the coating film in such a manner that the skinning which may occur upon drying from the coating surface side is prevented, e.g., by heating the coating film from the release film (release member) side.

By appropriately selecting the kinds of the constituent ingredients, combination, proportions, etc. of an aqueous polymer dispersion for forming a supporting layer and an aqueous polymer dispersion for forming a pressure-sensitive adhesive layer and by producing a pressure-sensitive adhesive sheet by process (A) and/or process (B), then the components of the resultant supporting layer are mixed with those of the resultant pressure-sensitive adhesive layer at the boundary between these layers and a satisfactorily anchored state can be realized. In this method, a drying step is carried out once and a supporting layer and a pressure-sensitive adhesive layer can be simultaneously formed as united layers. Consequently, this method is effective in simplifying a process and improving the working efficiency.

A section of the pressure-sensitive adhesive sheet for medical use of the invention was examined with a transmission electron microscope (TEM) at a magnification of 50,000 diameters. As a result, it was ascertained that the boundary between the supporting layer and the pressure-sensitive adhesive layer included an area in which the two layers were mixed with each other.

Furthermore, since each of the layers constituting the pressure-sensitive adhesive sheet of the invention has been formed from an aqueous polymer dispersion without using an organic solvent, the production of this adhesive sheet does not cause an environmental problem and is free from troubles such as the swelling or dissolution of the lower layer during application and drying. A satisfactory workability is hence attained in the formation of the supporting layer and pressure-sensitive adhesive layer. In addition, a residual monomer is not formed during the formation of each layer.

In addition, the pressure-sensitive adhesive sheet thus produced is excellent in adhesive properties such as adhesive force and holding power and the properties of the supporting layer can be arbitrarily modified. Thus, the performances of the whole sheet can be regulated easily. When the sheet has a supporting layer and a pressure-sensitive adhesive layer provided on each side thereof, it functions as a double-faced adhesive sheet and the adhesive properties thereof can be modified by regulating the material composition and thickness of each pressure-sensitive adhesive layer. Furthermore, by changing the physical properties and thickness of the supporting layer, the range of property modification is widened and the adhesive sheet can be used in various applications. As in the case of the double-faced adhesive sheet, the sheet comprising a supporting layer and a pressure-sensitive adhesive layer provided on only one side thereof also can be made to have a performance which makes the sheet suitable for use in a particular application, by modifying the adhesive properties thereof or regulating the physical properties of the supporting layer.

As the release member, any of papers, laminated papers, various plastic films, metal foils, and the like which each have undergone a silicone treatment on at least one side may be used. The release member to be used may have any desired shape such as a sheet, tape, or belt form. After a multilayer structure comprising a pressure-sensitive adhesive layer and a supporting layer is formed, the release member may be peeled off and removed. Alternatively, the release member may be kept adherent as a release liner in order to protect the pressure-sensitive adhesive layer until use.

The thickness of each layer in the pressure-sensitive adhesive sheet is not particularly limited, and preferably the thickness is suitably set according to the intended use or purpose. For example, the thickness of the supporting layer is preferably in the range of from 10 to 150 µm and that of the pressure-sensitive adhesive layer is preferably in the range of from 10 to 150 µm. Cushioning properties may be imparted to the supporting layer by adding a foaming agent to the aqueous polymer dispersion for forming a supporting layer and foaming the coating layer of the dispersion after application. In this case, the thickness of the supporting layer may be in the range of from 50 to 2,000 µm.

The invention will be explained below in detail by reference to Examples, but the invention should not be construed as being limited thereto. In the following Examples, all parts are by weight.

### (EXAMPLE 1)

### <Preparation of Aqueous Polymer Dispersion for Forming a Supporting Layer>

To 100 parts of polypropylene glycol having a number-average molecular weight of 3,000 was added a monomer mixture composed of 45 parts of butyl acrylate, 45 parts of ethyl acrylate, and 10 parts of acrylic acid. Then, 2 parts of 2-mercaptoethanol as a chain transfer agent having a hydroxyl group and 0.1 part of 2,2-azobisisobutyronitrile as a polymerization initiator were added. A polymerization reaction was carried out at 60°C for 4 hours in a nitrogen stream to obtain a viscous liquid comprising a mixture of the polypropylene glycol and an acrylic polymer having a number-average molecular weight of 7,500.

To this viscous liquid was added 23.5 parts of isophorone diisocyanate (2.3 equivalents to all hydroxyl groups). This mixture was subjected to reaction at 65°C for 3 hours to synthesize an isocyanate prepolymer. To this isocyanate prepolymer was added 14 parts of triethylamine (one equivalent to the carboxyl groups) to neutralize the carboxyl groups. Thereafter, 600 parts of water was added thereto with stirring to disperse the isocyanate prepolymer in the water. Subsequently, a solution prepared by diluting 1.8 parts of ethylenediamine (1/2 equivalent to the residual isocyanate groups) with 16.2 parts of water was added to the dispersion. This mixture was subjected to reaction at 65°C for 3 hours to extend the main chain.

A monomer for imparting nontackiness, which contains 113.7 parts of isobornyl acrylate (glass transition temperature of the polymer, 367 K) was added, with stirring, to the aqueous urethane-acrylic composite dispersion obtained above to thereby cause the urethane-acrylic core polymer particles contained in the aqueous urethane-acrylic composite dispersion to absorb the monomer for imparting nontackiness. Subsequently, 0.11 part of 2,2-azobis[2-(2-imidazolin-2-yl)]propane was added thereto to initiate a polymerization reaction. This mixture was held at 60°C for 4 hours, subsequently heated to 70°C and held at this temperature for 1 hour, and then cooled. This polymerization treatment gave an aqueous urethane-acrylic composite dispersion which contains water and, stably dispersed therein, a urethane-acrylic polymer having nontackiness at room temperature and comprising 29% by weight polyol components, 7% by weight polyisocyanate components, and 63% by weight acrylic components (the remainder contains the neutralizing agent, the urethane main chain extending agent, etc.).

This aqueous urethane-acrylic composite dispersion was used as an aqueous polymer dispersion for forming a supporting layer.

### Coating Film Properties of the Aqueous Polymer Dispersion for Forming a Supporting Layer:

The aqueous urethane-acrylic composite dispersion was applied to a releasability-imparted polyester film and dried at 110°C for 5 minutes to form a film having a uniform thickness (thickness, 50 µm). The film obtained was subjected to the tensile tests shown below to determine the 100% modulus and the stress relaxation time. As a result, the film was found to have a 100% modulus of 5 N/mm² and a stress relaxation time of 22 seconds.

### (1) 100% Modulus

A test piece was produced so as to have a cross-sectional area of about 1 mm² and a length of 20 mm. This test piece was subjected to a tensile test with a tensile tester ("Autograph Type AGS-50D" manufactured by Shimadzu Corp.) at a tensile rate of 300 mm/min. The 100% modulus of the test piece was read from the stress-strain curve thus obtained.

### (2) Stress Relaxation Time

A test piece was produced so as to have a cross-sectional area of about 1 mm² and a length of 20 mm. This test piece was pulled with a tensile tester ("Autograph Type AGS-50D" manufactured by Shimadzu Corp.) at a tensile rate of 300 mm/min. At the time when the test piece had been elongated by 50% based on the original test piece length (i.e., by 10 mm), the tensile tester was stopped. The stress at this time was taken as initial stress, and the time period required for this initial stress to decrease to 1/e (e=2.7183) was taken as the stress relaxation time.

### <Preparation of Aqueous Polymer Dispersion For Forming a Pressure-Sensitive Adhesive Layer>

A monomer mixture of 95 parts of 2-ethylhexyl acrylate and 5 parts of acrylic acid was emulsion-polymerized in an ordinary manner using 2 parts of sodium lauryl sulfate as an emulsifying agent and 0.2 parts of potassium persulfate as a polymerization initiator. Thus, an aqueous dispersion having a solid concentration of 50% by weight was obtained which contains water and an acrylic polymer homogeneously emulsified and dispersed therein. This acrylic polymer had an intraparticulate gel content (crosslinked component content) of 46% by weight, and the solvent-soluble components of the polymer had a weight-average molecular weight of 800,000. This dispersion was used as an aqueous polymer dispersion for forming a pressure-sensitive adhesive layer.

### <Production of Pressure-Sensitive Adhesive Tape>

A polyethylene-laminated paper having a thickness of 38 µm and having a surface treated with a silicone was coated on the silicone-treated side simultaneously with the aqueous polymer dispersion for forming a pressure-sensitive adhesive layer and the aqueous polymer dispersion for forming a supporting layer using a die coater having two-layer manifolds. Thereafter, the whole coated paper was dried at 90°C for 5 minutes and then at 120°C for 1 minute to obtain a pressure-sensitive adhesive tape which has a release liner and, provided thereon, a pressure-sensitive adhesive layer and a supporting layer. The thickness of the pressure-sensitive adhesive layer after drying was 50 µm, and the thickness of the supporting layer after drying was 50 µm.

### (EXAMPLE 2)

### <Preparation of Aqueous Polymer Dispersion for Forming a Supporting Layer>

To 100 parts of polytetramethylene glycol having a number-average molecular weight of 3,000 was added a monomer mixture of 45 parts of butyl acrylate, 20 parts of methyl methacrylate, and 10 parts of acrylic acid. Then, 2 parts of 2-mercaptoethanol as a chain transfer agent having a hydroxyl group and 0.1 part of 2,2-azobisisobutyronitrile as a polymerization initiator were added. A polymerization reaction was carried out at 65°C for 7 hours in a nitrogen stream to obtain a viscous liquid comprising a mixture of the polytetramethylene glycol and an acrylic polymer having a number-average molecular weight of 7,400.

To this viscous liquid were added 30.6 parts of 4,4-dicyclohexylmethane diisocyanate and 4.5 parts of xylylene diisocyanate (3 equivalents to all hydroxyl groups). This mixture was subjected to reaction at 65°C for 3 hours to synthesize an isocyanate prepolymer. To this isocyanate prepolymer was added 14 parts of triethylamine (one equivalent to the carboxyl groups) to neutralize the carboxyl groups. Thereafter, 630 parts of water was added thereto with stirring to disperse the isocyanate prepolymer in the water. Subsequently, a solution prepared by diluting 2.8 parts of ethylenediamine (one equivalent to the residual isocyanate groups) with 24.2 parts of water was added to the dispersion. This mixture was subjected to reaction at 65°C for 3 hours to extend the main chain.

A monomer mixture for imparting nontackiness which contains 10 parts of butyl acrylate and 50 parts of methyl methacrylate (glass transition temperature of the polymer, 337 K) was added, with stirring, to the aqueous urethane-acrylic composite dispersion obtained above. This mixture' was stirred for 1 hour in a nitrogen stream to thereby cause the urethane-acrylic core polymer particles contained in the aqueous urethane-acrylic composite dispersion to absorb the nontackiness-imparting monomer mixture. Subsequently, the dispersion was heated to 65°C and 0.06 parts of 2,2-azobis[2-(2-imidazolin-2-yl)]propane was added thereto to initiate a polymerization reaction. This mixture was held at 65°C for 4 hours, subsequently heated to 80°C and held at this temperature for 1 hour, and then cooled. Through this polymerization treatment, an aqueous urethane-acrylic composite dispersion was obtained which contains water and, stably dispersed therein, a urethane-acrylic polymer having no tackiness at room temperature and comprising 33% by weight polyol components, 10% by weight polyisocyanate components, and 54% by weight acrylic components (the remainder contains the neutralizing agent, urethane main chain extender, etc.).

This aqueous urethane-acrylic composite dispersion was used as an aqueous polymer dispersion for forming a supporting layer.

### Coating Film Properties of the Aqueous Polymer Dispersion for Forming a Supporting Layer:

The aqueous urethane-acrylic composite dispersion was applied to a releasability-imparted polyester film and dried at 110°C for 3 minutes to form a film having a uniform thickness (thickness, 50 µm). The film obtained was subjected to tensile tests in the same manner as in Example 1 to determine the 100% modulus and the stress relaxation time.

As a result, the film was found to have a 100% modulus of 8.0 N/mm² and a stress relaxation time of 30 seconds.

### <Preparation of Aqueous Polymer Dispersion for Forming a Pressure-Sensitive Adhesive Layer>

To 100 parts of a diethylene glycol adipic acid ester (number-average molecular weight: 2,500; hydroxyl value, 41) was added 49.2 parts of 4,4-dicyclohexylmethane diisocyanate. This mixture was reacted at 65°C for 1 hour and then cooled to 30°C. Thereafter, 12.4 parts of dimethylolpropionic acid was dissolved in 31 parts of N-methylpyrrolidone and this solution was added thereto. The resultant mixture was degassed and then subjected to reaction at 70°C for 3 hours to obtain a urethane prepolymer having carboxyl groups.

To the urethane prepolymer obtained was added 1,454.4 parts of butyl acrylate (urethane prepolymer/butyl acrylate weight ratio, 10/90). This mixture was sufficiently stirred. Furthermore, 9.4 parts of triethylamine was added thereto and this mixture was sufficiently stirred for neutralization. Distilled water in an amount of 2,762.4 parts was introduced into another flask, and nitrogen displacement was carried out for 1.5 hours. Thereafter, the mixture neutralized was added dropwise using a dropping funnel. After completion of this dropwise addition, 4 parts of ethylenediamine diluted by three times with distilled water was added thereto, followed by 2.19 parts of azobisisobutyronitrile dissolved in 6 parts of N-methylpyrrolidone. The resultant mixture was heated to 60°C and reacted for 2 hours to complete chain extension and polymerization. The aqueous urethane-acrylic composite dispersion obtained was thickened with a polycarboxylic acid type thickener to obtain an aqueous dispersion. This dispersion was used as an aqueous polymer dispersion for forming a pressure-sensitive adhesive layer.

### <Production of Pressure-Sensitive Adhesive Tape>

A polyethylene-laminated paper having a thickness of 38 µm and having a surface treated with a silicone (release liner) was coated on the silicone-treated side simultaneously with the aqueous polymer dispersion for forming a pressure-sensitive adhesive layer and the aqueous polymer dispersion for forming a supporting layer using a die coater having two-layer manifolds. Thereafter, the whole release liner was dried at 120°C for 3 minutes to obtain a pressure-sensitive adhesive tape which has a release liner and, provided thereon, a pressure-sensitive adhesive layer and a supporting layer. The thickness of the pressure-sensitive adhesive layer after drying was 70 µm, and the thickness of the supporting layer after drying was 70 µm.

### (EXAMPLE 3)

The aqueous polymer dispersion for forming a supporting layer and the aqueous polymer dispersion for forming a pressure-sensitive adhesive layer both prepared in Example 1 were used. A mixture obtained by adding 100 parts of sorbitan trioleate as a plasticizer to 100 parts on a solid basis of the aqueous polymer dispersion for forming a pressure-sensitive adhesive layer was applied to the same release liner as in Example 1 in a thickness of 50 µm on a dry basis. Subsequently, the aqueous polymer dispersion for forming a supporting layer was applied thereon with a dispenser in a thickness of 50 µm on a dry basis. Thereafter, the whole release liner coated was dried at 80°C for 10 minutes and then at 120°C for 1 minute. Thus, a pressure-sensitive adhesive tape was obtained which has the release liner and, superposed thereon in this order, a pressure-sensitive adhesive layer and a supporting layer.

### (EXAMPLE 4)

The aqueous polymer dispersion for forming a supporting layer prepared in Example 2 was used. A natural rubber latex was further used as an aqueous polymer dispersion for forming a pressure-sensitive adhesive layer. The aqueous polymer dispersion for forming a pressure-sensitive adhesive layer was applied to the same release liner as in Example 1. Then, the aqueous polymer dispersion for forming a supporting layer and the aqueous polymer dispersion for forming a pressure-sensitive adhesive layer with a die coater having two manifolds. Thereafter, the whole was dried at 90°C for 5 minutes and then at 120°C for 1 minute. Thus, a double-faced pressure-sensitive adhesive tape was obtained which has the release liner and, superposed thereon in this order, a pressure-sensitive adhesive layer, a supporting layer, and a pressure-sensitive adhesive layer. The thicknesses of these layers after drying were as follows: pressure-sensitive adhesive layer/supporting layer/pressure-sensitive adhesive layer = 30 µm/50 µm/30 µm.

### (COMPARATIVE EXAMPLE 1)

The aqueous polymer dispersion for forming a supporting layer and the aqueous polymer dispersion for forming a pressure-sensitive adhesive layer both prepared in Example 1 were used. The aqueous polymer dispersion for forming a pressure-sensitive adhesive layer was applied to the same release liner as in Example 1 in a thickness of 50 µm on a dry basis and dried at 110°C for 3 minutes. The aqueous polymer dispersion for forming a supporting layer was applied to another release liner in a thickness of 70 µm on a dry basis and dried at 110°C for 3 minutes. Thereafter, these films dried were laminated to each other to produce a pressure-sensitive adhesive tape.

### (COMPARATIVE EXAMPLE 2)

A pressure-sensitive adhesive tape was produced in the same manner as in Comparative Example 1, except that the aqueous polymer dispersion for forming a supporting layer and the aqueous polymer dispersion for forming a pressure-sensitive adhesive layer both prepared in Example 3 were used in producing the pressure-sensitive adhesive tape.

### (COMPARATIVE EXAMPLE 3)

A commercial poly(vinyl chloride) film (thickness, 70 µm) was used as a supporting layer and the aqueous polymer dispersion for forming a pressure-sensitive adhesive layer prepared in Example 2 was used as an aqueous polymer dispersion for forming a pressure-sensitive adhesive layer to produce a pressure-sensitive adhesive tape. Namely, the aqueous polymer dispersion for forming a pressure-sensitive adhesive layer was applied to the poly(vinyl chloride) film in a thickness of 30 µm on a dry basis and dried at 110°C for 3 minutes to produce a pressure-sensitive adhesive tape.

### (COMPARATIVE EXAMPLE 4)

A pressure-sensitive adhesive tape was produced in the same manner as in Comparative Example 1, except that the aqueous polymer dispersion for forming a supporting layer and the aqueous polymer dispersion for forming a pressure-sensitive adhesive layer both prepared in Example 4 were used.

### (EVALUATION OF ANCHORING)

The pressure-sensitive adhesive tapes obtained in Examples 1 to 4 and Comparative Examples 1 to 4 each were evaluated for anchoring by the following methods.

### <Adhesive Remaining After Stripping from Skin>

The pressure-sensitive adhesive sheet cut into a rectangular shape of 20 mm by 60 mm was applied to the back of a volunteer who had lain quietly for at least 30 minutes in a test chamber having a constant temperature of 23°C and a constant relative humidity of 30%. This application was accomplished by contact-bonding of the adhesive sheet by rolling a 2-kg roller forward and backward once on the sheet. At 2 hours after the application (adhesive remaining evaluation just after production) or at 3 days after the application (adhesive remaining evaluation after 3 days), the pressure-sensitive adhesive sheet was stripped off in the 180° direction at a peeling rate of 300 mm/min. After the stripping, the skin surface was visually examined for adhesive remaining. The degree of adhesive remaining on the skin surface was judged based on the following criteria. The results obtained are shown in Table 1.

### Criteria for Judgement:

A: No adhesive remaining on the skin surface was observed.
B: Adhesive remaining was observed on part of the skin surface.
C: Adhesive remaining was observed on the whole skin surface.

### <Confirmation of Anchoring between Supporting Layer and Pressure-Sensitive Adhesive Layer>

The boundary between the supporting layer and pressure-sensitive adhesive layer in a section of the pressure-sensitive adhesive sheet was examined with a transmission electron microscope (TEM) at a magnification of 50,000 diameters. The case in which the boundary between the supporting layer and the pressure-sensitive adhesive layer included an area in which the two layers were mingled with each other is indicated by symbol "A", while the case in which the boundary included no area in which the supporting layer and the pressure-sensitive adhesive layer were mingled with each other is indicated by the symbol "B". The results obtained are shown in Table 1.

**Table 1**

| | | Example | | | | Comparative Example | | | |
|---|---|---|---|---|---|---|---|---|---|
| | | 1 | 2 | 3 | 4 | 1 | 2 | 3 | 4 |
| Adhesive remaining | Immediately after production | A | A | A | A | B | C | C | C |
| | After 3 days | A | A | A | A | A | C | C | C |
| Presence of area in which supporting layer mingled with pressure-sensitive adhesive layer | | A | A | A | A | B | B | B | B |

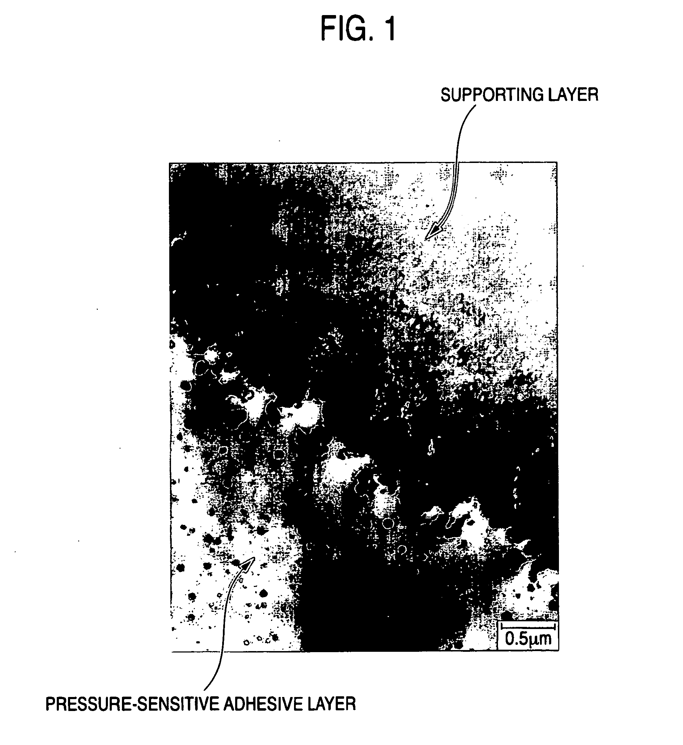

Table 1 shows the following. From transmission electron photomicrographs, the pressure-sensitive adhesive sheets of the invention obtained in Examples 1 to 4 were confirmed to have, at the boundary between the supporting layer and the pressure-sensitive adhesive layer, an area in which the two layers were mingled with each other. Even immediately after the production, these adhesive sheets did not show adhesive remaining at all and had satisfactory anchoring. Fig. 1 shows a transmission electron photomicrograph (50,000 diameters) of a section of the pressure-sensitive adhesive sheet of Example 1. As apparent from Fig. 1, the boundary between the supporting layer and the pressure-sensitive adhesive layer was confirmed to include an area in which the two layers were mingled with each other. Since the production of the pressure-sensitive adhesive sheets of Examples 1 to 4 requires only one drying step, process simplification and improvement in working efficiency can be achieved. Furthermore, the pressure-sensitive adhesive sheets of Examples 1 to 4 were flexible and had satisfactory stress relaxation characteristics.

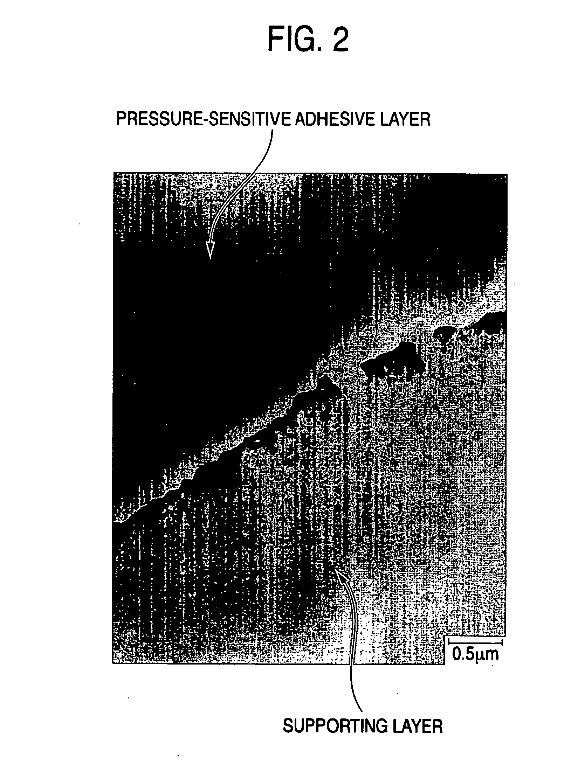

In contrast, the pressure-sensitive adhesive sheet obtained in Comparative Example 1 showed adhesive remaining immediately after production and was hence unable to be processed immediately after production. Furthermore, the production process in Comparative Example 1 involved many steps because the two layers were formed separately and then laminated to each other. This process hence had a poor working efficiency. Fig. 2 shows a transmission electron photomicrograph (50,000 diameters) of a section of the pressure-sensitive adhesive sheet of Comparative Example 1. At the boundary between the supporting layer and the pressure-sensitive adhesive layer, no area was observed in which the two layers were mingled with each other. In the pressure-sensitive adhesive sheets obtained in Comparative Examples 2 to 4, the boundary between the supporting layer and the pressure-sensitive adhesive layer included no area in which the two layers were mingled with each other. These adhesive sheets showed adhesive remaining immediately after production and after 3 days. They were hence unsatisfactory in anchoring.

As described above in detail, the invention can provide a pressure-sensitive adhesive sheet for medical use which realizes satisfactory anchoring even immediately after production and can be produced without necessitating the use of a primer, which is required to be properly selected, or requiring a priming step, which leads to a cost increase. The production of this adhesive sheet does not pose an environmental or another problem attributable to an organic solvent or the like. The adhesive sheet can be produced at an improved efficiency. The invention can further provide processes for producing the adhesive sheet.

## Claims

1. A process for producing a pressure-sensitive adhesive sheet for medical use, which comprises simultaneously applying at least a coating fluid for forming a pressure-sensitive adhesive layer and a coating fluid for forming a supporting layer in this order to a releasability-imparted side of a release member and then drying the coating fluids to form a multilayer structure comprising a pressure-sensitive adhesive layer and a supporting layer which are in the state of being anchored to each other at the boundaries thereof.

2. A process for producing a pressure-sensitive adhesive sheet for medical use, which comprises applying at least a coating fluid for forming a pressure-sensitive adhesive layer to a releasability-imparted side of a release member, subsequently applying a coating fluid for forming a supporting layer on the pressure-sensitive adhesive layer before the pressure-sensitive adhesive layer dries, and then drying the whole to thereby form a multilayer structure comprising a supporting layer and a pressure-sensitive adhesive layer which are in the state of being anchored each other at the boundary thereof.

3. A pressure-sensitive adhesive sheet for medical use, which comprises a supporting layer and a pressure-sensitive adhesive layer provided on at least one side of the supporting layer, wherein each of the supporting layer and the pressure-sensitive adhesive layer is prepared from an aqueous polymer dispersion and the supporting layer and the pressure-sensitive adhesive layer are in the state of being anchored each other at the boundary thereof, the sheet being obtainable by the process of claim 1 or 2.

4. The pressure-sensitive adhesive sheet for medical use of claim 3, wherein the aqueous polymer dispersion used for forming the supporting layer is an aqueous urethane -acrylic composite dispersion.

5. The pressure-sensitive adhesive sheet of claim 3, wherein the aqueous polymer dispersion for forming the supporting layer is capable of giving, after application and drying, a coating film having a 100% modulus of from 5 N/mm² to 15 N/mm².

6. The pressure-sensitive adhesive sheet of claim 4, wherein the aqueous polymer dispersion for forming the supporting layer is capable of giving, after application and drying, a coating film having a 100% modulus of from 5 N/mm² to 15 N/mm².

7. The pressure-sensitive adhesive sheet of claim 3, wherein the aqueous polymer dispersion for forming the supporting layer is capable of giving, after application and drying, a coating film having a stress relaxation time of 50 seconds or shorter.

8. The pressure-sensitive adhesive sheet of claim 4, wherein the aqueous polymer dispersion for forming the supporting layer is capable of giving, after application and drying, a coating film having a stress relaxation time of 50 seconds or shorter.

9. The pressure-sensitive adhesive sheet of claim 5, wherein the aqueous polymer dispersion for forming the supporting layer is capable of giving, after application and drying, a coating film having a stress relaxation time of 50 seconds or shorter.

10. The pressure-sensitive adhesive sheet of claim 6, wherein the aqueous polymer dispersion for forming the supporting layer is capable of giving, after application and drying, a coating film having a stress relaxation time of 50 seconds or shorter.

## Patentansprüche

1. Verfahren zur Herstellung einer druckempfindlichen Klebefolie für medizinische Zwecke, umfassend das gleichzeitige Auftragen von mindestens einer Beschichtungsflüssigkeit zur Bildung einer druckempfindlichen Klebeschicht und einer Beschichtungsflüssigkeit zur Bildung einer Unterstützungsschicht in dieser Reihenfolge auf die Seite einer Ablösungseinheit, die mit Ablösungseigenschaften versehen ist, und anschließendes Trocknen der Beschichtungsflüssigkeiten zur Bildung einer Mehrschichtstruktur, welche eine druckempfindliche Klebeschicht und eine Unterstützungsschicht umfasst, die an ihren Grenzen ineinander verankert sind.

2. Verfahren zur Herstellung einer druckempfindlichen Klebefolie für medizinische Zwecke, umfassend das Auftragen mindestens einer Beschichtungsflüssigkeit zur Bildung einer druckempfindlichen Klebeschicht auf eine Seite einer Ablösungseinheit, welche Ablösungseigenschaften aufweist, anschließendes Auftragen einer Beschichtungsflüssigkeit zur Bildung einer Unterstützungsschicht auf der druckempfindlichen Klebeschicht, bevor die druckempfindliche Klebeschicht getrocknet ist, und dann Trocknen der so gebildeten Mehrschichtstruktur, welche eine Unterstützungsschicht und eine druckempfindliche Klebeschicht umfasst, die an ihren Grenzen ineinander verankert sind.

3. Druckempfindliche Klebefolie für medizinische Zwecke, umfassend eine Unterstützungsschicht sowie eine druckempfindliche Klebeschicht, welche sich auf mindestens einer Seite der Unterstützungsschicht befindet, worin die Unterstützungsschicht und die druckempfindlichen Klebeschicht aus einer wässrigen Polymerdispersion hergestellt wurden, wobei die Unterstützungsschicht und die druckempfindliche Klebeschicht an ihren Grenzen ineinander verankert sind, und wobei die Folie erhältlich ist durch das Verfahren gemäß Anspruch 1 oder 2.

4. Druckempfindliche Klebefolie für medizinische Zwecke gemäß Anspruch 3, worin die wässrige Polymerdispersion, welche zur Bildung der Unterstützungsschicht verwendet wird, eine wässrige Urethan-Acryl-Kompositdispersion ist.

5. Druckempfindliche Klebefolie gemäß Anspruch 3, worin die wässrige Polymerdispersion zur Bildung der Unterstützungsschicht in der Lage ist, nach dem Auftragen und Trocknen einen Filmüberzug zu bilden, welcher einen 100% Modul von 5 N/mm² bis 15 N/mm² hat.

6. Druckempfindliche Klebefolie gemäß Anspruch 4, worin die wässrige Polymerdispersion zur Bildung der Unterstützungsschicht in der Lage ist, nach dem Auftragen und Trocknen einen Filmüberzug zu bilden, welcher einen 100% Modul von 5 N/mm² bis 15 N/mm² hat.

7. Druckempfindliche Klebefolie gemäß Anspruch 3, worin die wässrige Polymerdispersion zur Bildung der Unterstützungsschicht in der Lage ist, nach dem Auftragen und Trocknen einen Filmüberzug zu bilden, welcher eine Spannungsrelaxationszeit von 50 Sekunden oder weniger hat.

8. Druckempfindliche Klebefolie gemäß Anspruch 4, worin die wässrige Polymerdispersion zur Bildung der Unterstützungsschicht in der Lage ist, nach dem Auftragen und Trocknen einen Filmüberzug zu bilden, welcher eine Spannungsrelaxationszeit von 50 Sekunden oder weniger hat.

9. Druckempfindliche Klebefolie gemäß Anspruch 5, worin die wässrige Polymerdispersion zur Bildung der Unterstützungsschicht in der Lage ist, nach dem Auftragen und Trocknen einen Filmüberzug zu bilden, der eine Spannungsrelaxationszeit von 50 Sekunden oder weniger hat.

10. Druckempfindliche Klebefolie gemäß Anspruch 6, worin die wässrige Polymerdispersion zur Bildung einer Unterstützungsschicht in der Lage ist, nach dem Auftragen und Trocknen einen Filmüberzug zu bilden, welcher eine Spannungsrelaxationszeit von 50 Sekunden oder weniger hat.

## Revendications

1. Procédé de production d'une feuille adhésive sensible à la pression à usage médical, qui comprend l'application simultanée d'au moins un fluide de revêtement pour former une couche adhésive sensible à la pression et un fluide de revêtement pour former une couche de soutien dans cet ordre à un côté auquel on a donné une aptitude au décollage d'un élément à décollage multiple puis le séchage des fluides de revêtement pour former une structure multicouche comprenant une couche adhésive sensible à la pression et une couche de soutien qui sont ancrées l'une à l'autre au niveau de leurs limites.

2. Procédé de production d'une feuille adhésive sensible à la pression à usage médical, qui comprend l'application d'au moins un fluide de revêtement pour former une couche adhésive sensible à la pression à un côté auquel on a donné une aptitude au décollage d'un élément à décollage multiple, l'application ultérieure d'un fluide de revêtement pour former une couche de soutien sur la couche adhésive sensible à la pression avant que la couche adhésive sensible à la pression ne sèche, puis le séchage de l'intégralité pour former ainsi une structure multicouche comprenant une couche de soutien et une couche adhésive sensible à la pression qui sont ancrées l'une à l'autre au niveau de leurs limites.

3. Feuille adhésive sensible à la pression à usage médical, qui comprend une couche de soutien et une couche adhésive sensible à la pression prévue sur au moins un côté de la couche de soutien, dans laquelle chacune de la couche de soutien et de la couche adhésive sensible à la pression est préparée à partir d'une dispersion aqueuse de polymères et la couche de soutien et la couche adhésive sensibles à la pression sont ancrées l'une à l'autre au niveau de leurs limites, la feuille pouvant être obtenue par le procédé selon la revendication 1 ou 2.

4. Feuille adhésive sensible à la pression à usage médical selon la revendication 3, dans laquelle la dispersion aqueuse de polymères pour former la couche de soutien est une dispersion aqueuse composite uréthane-acrylique.

5. Feuille adhésive sensible à la pression selon la revendication 3, dans laquelle la dispersion aqueuse de polymères pour former la couche de soutien est capable de donner, après application et séchage, un film de revêtement ayant un module de 100 % de 5 N/mm² à 15 N/MM².

6. Feuille adhésive sensible à la pression selon la revendication 4, dans laquelle la dispersion aqueuse de polymères pour former la couche de soutien est capable de donner, après application et séchage, un film de revêtement ayant un module de 100 % de 5 N/mm² à 15 N/mm².

7. Feuille adhésive sensible à la pression selon la revendication 3, dans laquelle la dispersion aqueuse de polymères pour former la couche de soutien est capable de donner après application et séchage, un film de revêtement ayant un temps de relaxation de contrainte de 50 secondes ou moins.

8. Feuille adhésive sensible à la pression selon la revendication 4, dans laquelle la dispersion aqueuse de polymères pour former la couche de soutien est capable de donner, après application et séchage, un film de revêtement ayant un temps de relaxation de contrainte de 50 secondes ou moins.

9. Feuille adhésive sensible à la pression selon la revendication 5, dans laquelle la dispersion aqueuse de polymères pour former la couche de soutien est capable de donner, après application et séchage, un film de revêtement ayant un temps de relaxation de contrainte de 50 secondes ou moins.

10. Feuille adhésive sensible à la pression selon la revendication 6, dans laquelle la dispersion aqueuse de polymères pour former la couche de soutien est capable de donner, après application et séchage, un film de revêtement ayant un temps de relaxation de contrainte de 50 secondes ou moins.
